# EUROPÄISCHE PATENTSCHRIFT

(11) **EP 3 419 547 B1**
(45) Veröffentlichungstag und Bekanntmachung des Hinweises auf die Patenterteilung: **28.04.2021**
(21) Anmeldenummer: 17707501.7
(22) Anmeldetag: 21.02.2017
(51) Int. Cl.: A61B 50/30, A61L 2/07, A61L 2/26, A61B 50/00

(54) **STERILBEHÄLTER MIT EINER GASDURCHLÄSSIGEN FILTERKASETTE.**
STERILE CONTAINER WITH GAS-PERMEABLE FILTER CASSETTE.
CONTENEUR DE STÉRILISATION AVEC CASSETTE FILTRANTE PERMÉABLE AUX GAZ.

(30) Priorität: 25.02.2016 DE 102016103342
(43) Veröffentlichungstag der Anmeldung: 02.01.2019
(73) Patentinhaber: Aesculap AG, 78532 Tuttlingen (DE)
(72) Erfinder: BELLIKLI, Serkan, 72355 Schömberg (DE); GRAY-DREIZLER, John, 78078 Niedereschach (DE); ELISCH, Andreas, 78713 Schramberg (DE); WEISS, Josef-Benedikt, 78628 Rottweil (DE)
(74) Vertreter: Winter, Brandl - Partnerschaft mbB
(86) Internationale Anmeldenummer: PCT/EP2017/053913
(87) Internationale Veröffentlichungsnummer: WO 2017/144453

(56) Entgegenhaltungen:
- WO-A1-03/041604
- GB-A- 908 407
- US-A1- 2005 045 551

## Beschreibung

Die vorliegende Erfindung betrifft einen Sterilbehälter mit einer gasdurchlässigen Filterkassette.

Bei der überwiegenden Mehrheit klinischer Handlungen und Operationen ist die Gewährleistung der Sterilität der verwendeten Instrumente und / oder sonstiger Hilfsmittel unerlässlich. Aus diesem Grund werden Sterilcontainer /-behälter (auch als Sterilisationscontainer /-behälter bezeichnet) verwendet, welche beispielsweise mit medizinischen Instrumenten bestückt / befüllt werden. Daraufhin wird der Sterilbehälter im bestückten Zustand beispielsweise in einem Autoklaven für eine vorherbestimmte Zeitdauer auf eine vorherbestimmte Sterilisationstemperatur erhitzt, bis etwaige an den medizinischen Instrumenten anhaftende Mikroorganismen abgetötet sind.

Dabei ist ein Sterilfilter erforderlich, um nach dem Sterilisationsvorgang das Eindringen von Keimen in den Aufnahmeraum des Sterilbehälters zu verhindern. Da der Gasdurchsatz durch einen Sterilfilter begrenzt ist, kann zusätzlich ein Überdruckventil in dem Sterilbehälter vorgesehen sein, um eine große Druckdifferenz zwischen dem Behälter-Äußeren und dem Behälter-Inneren auszugleichen und eine Beschädigung des Sterilbehälters zu verhindern. Solch eine große Druckdifferenz tritt beispielsweise bei der Sterilisation des Behälterguts mittels gesättigtem Wasserdampf auf. Aus dem Stand der Technik sind bereits verschiedene Ausführungsformen von Filterkassetten und Sterilbehältern mit solchen Filterkassetten bekannt, welche zum Einen den Inhalt des Sterilbehälters nach der Sterilisation vor einer Verunreinigung von Keimen schützen und zum Anderen einen weiteren Strömungspfad im Falle einer hohen Druckdifferenz zwischen dem Behälter-Äußeren und dem Behälter-Inneren freigegeben.

Beispielsweise ist aus der DE 88 14 071 U1 ein Sterilbehälter bekannt, bei dem ein an einem Sterilfilter vorbeiführender Strömungspfad bei Überschreiten einer vorbestimmten Druckdifferenz freigegeben wird. Dafür werden in der Ausgangsposition eine Abdeckscheibe und eine Filterscheibe durch Schraubenfedern von einer Abdeckung weggedrückt und ein Gasaustausch zwischen der Umgebung und dem Aufnahmeraum ist nur durch die Filterscheibe möglich. Bei Überschreiten der vorbestimmten Druckdifferenz werden die Abdeckscheibe und die Filterscheibe in Richtung der Abdeckung verschoben und über mehrere Silikondichtungen gegenüber dem eintretenden Gas geschützt, welches einen aufgrund der Verschiebung freigegebenen Strömungspfad benutzt, um in den Aufnahmeraum einzudringen. Die Abdeckscheibe und die Filterscheibe sind dabei über einen Lagerzapfen zentriert und müssen entsprechend zusätzlich abgedichtet werden. Die in der DE 88 14 071 U1 offenbarten Konfiguration besteht somit aus vielen Einzelteilen, welche den Zusammenbau, das Handling und den Austausch des Filtersystems durch mehrere Handgriffe erschweren und das Gesamtgewicht der Ausführung erhöhen. Außerdem sind mehrere Dichtungen erforderlich, um eine Sterilbarriere sicherzustellen und aufgrund der Abdeckscheibe ist es nicht möglich, den Zustand des Filtermaterials von allen Seiten zu kontrollieren.

Zudem offenbart die DE 202 03 984 U1 einen Sterilbehälter mit einer Filtereinheit, welche sich aus einem Halterahmen, einem Filterelement und einer Abdeckung zusammensetzt. Die Teile sind dabei aus Kunststoff ausgebildet, was zu Problemen bei der Trocknung führt. Für den Zusammenbau muss der Halterahmen am Sterilbehälter und müssen das Filterelement und die Abdeckung am Halterahmen befestigt werden. Die Dichtung findet zwischen dem Filterelement und dem Sterilbehälter statt, die Anpresskraft wird jedoch über Rückstellfedern zwischen dem Sterilbehälter und dem Halterahmen erzeugt. Es ist daher schwierig eine sichere Dichtung zu gewährleisten, da die Anpresskraft nicht örtlich nahe der Dichtung eingeleitet wird.

Weitere Sterilbehälter mit Filtersystemen sind in der DE 10 2004 020 805 B3 und der DE 197 53 671 A1 beschrieben.

Vor diesem Hintergrund liegt der vorliegenden Erfindung die Aufgabe zugrunde, ein Sterilbarrieresystem zu entwickeln, welches bei einem einfachen Aufbau eine zuverlässige Funktionsweise gewährleistet.

Diese Aufgabe wird durch einen Sterilbehälter mit einer Filterkassette mit den Merkmalen des unabhängigen Anspruchs 1 gelöst. Vorteilhafte Weiterbildungen der Erfindung sind in den jeweiligen Unteransprüchen angegeben.

Eine gasdurchlässige Filterkassette des erfindungsgemäßen Sterilbehälters enthält ein in der Filterkassette angeordnetes Filterelement, wobei zum Zentrieren der Filterkassette bezüglich eines Sterilbehälterwandungsabschnitts, der zumindest eine Gasaustauschöffnung aufweist, an der Filterkassette ein erstes, kegelförmiges Zentriermittel vorgesehen ist, welches mit einem an dem Sterilbehälterwandungsabschnitt vorgesehenen zweiten Zentriermittel nichtselbsthemmend zusammenwirkt, wenn die Filterkassette in dem Sterilbehälterwandungsabschnitt (lose) eingesetzt oder an diesem angeordnet ist. Das erste Zentriermittel und das zweite Zentriermittel können an der Filterkassette bzw. an dem Sterilbehälterwandungsabschnitt jeweils mittig oder zentral angeordnet sein.

Mindestens ein Zentriermittel kann eine mittig zulaufende Abgleit- oder Anlagefläche aufweisen, welche bei einer Rückstellung von einer ausgelenkten Stellung in deren Anlageposition die Filterkassette zentral zur Gasaustauschöffnung positioniert.

Bei dem ersten Zentriermittel kann es sich insbesondere um einen kegelförmigen Vorsprung oder eine kegelförmige Aufnahme in der Filterkassette handeln, so dass die Filterkassette mit einer entsprechend komplementär ausgebildeten Aufnahme oder einem entsprechend komplementär ausgebildeten Vorsprung als das zweite Zentriermittel an dem Sterilbehälterwandungsabschnitt einen nicht-selbsthemmenden Kegelsitz bildet, wenn die Filterkassette in dem Sterilbehälterwandungsabschnitt eingesetzt oder an diesem angeordnet ist.

Durch die Zentrierung, insbesondere den Kegelsitz, kann die Filterkassette in den Sterilbehälterwandungsabschnitt eingesetzt werden, ohne dass, z.B. im Vergleich zu einer linearen Führung, die Gefahr eines Verkantens oder Verklemmens besteht. Es wird somit das Handling vereinfacht, da beim Einlegen der Filterkassette schnell und einfach die richtige Position insbesondere bezüglich der Gasaustauschöffnung im Sterilbehälterwandungsabschnitt gefunden wird. Außerdem muss aufgrund der Zentrierung mittels Kegelsitz keine zusätzliche durchgängige Aussparung oder dergleichen in der Filterkassette vorgesehen werden, was beispielsweise eine zusätzliche und aufwendige Dichtung mit sich führen würde. Es ist dabei sowohl möglich, den Kegelsitz über einen kegelförmigen Vorsprung in der Filterkassette und eine komplementär ausgebildete Aufnahme in dem Sterilbehälterwandungsabschnitt zu realisieren, als auch den Kegelsitz durch eine kegelförmige Aufnahme in der Filterkassette und einen komplementär ausgebildeten Vorsprung an dem Sterilbehälterwandungsabschnitt auszubilden.

Gemäß einem Aspekt der Erfindung kann die Filterkassette aus einer oberen Klemmscheibe und eine unteren Klemmscheibe ausgebildet sein.

Gemäß einem Aspekt der Erfindung kann das Filterelement zwischen der oberen Klemmscheibe und der unteren Klemmscheibe angeordnet sein.

Dies ermöglicht eine einfache Herstellung und Montage der Filterkassette, da die einzelnen Bauteile keine komplexen Formen ausbilden und in nur einem Fertigungsschritt miteinander verpresst werden können.

Dadurch ist das Filtermaterial, d.h. das Filterelement, durch die obere Klemmscheibe und die untere Klemmscheibe in einer Kassette eingefasst und es ist somit möglich das Filtermaterial vor Beschädigungen zu schützen.

Gemäß einem Aspekt der Erfindung kann ein Dichtring an der Filterkassette vorgesehen sein.

Gemäß einem Aspekt der Erfindung kann der Dichtring an der dem Sterilbehälterwandungsabschnitt zugewandten Seite der Filterkassette vorgesehen sein.

Gemäß einem Aspekt der Erfindung kann der Dichtring in einem äußeren Randabschnitt der Filterkassette angeordnet sein.

Der äußere Randbereich ist ein Bereich, welcher sich bündig an die Außenkante der Filterkassette, bzw. der unteren Klemmscheibe, anschließen kann. Er ist dabei auf jeden Fall so dimensioniert, dass der Dichtring die in dem Sterilbehälterwandungsabschnitt vorgesehene Gasaustauschöffnung vollständig umschließt. Es wird somit durch lediglich eine Dichtung im äußeren Randbereich der Filterkassette eine Dichtung zwischen dem Sterilbehälterwandungsabschnitt und der Filterkassette realisiert.

Gemäß einem Aspekt der Erfindung können die obere Klemmscheibe und die untere Klemmscheibe speichenradförmig ausgebildet sein.

Mit anderen Worten kann das Design der oberen Klemmscheibe und der untere Klemmscheibe an der Gestalt der Kieselalge angelehnt sein. Durch diesen bionischen Ansatz können das Gewicht und der Bauraum (insbesondere die Höhe) der Filterkassette bei gleichbleibenden mechanischen Eigenschaften optimiert werden.

Ein Filtersystem des erfindungsgemäßen Sterilbehälters enthält eine Filterkassette, eine Feder und eine an dem Sterilbehälterwandungsabschnitt montierbare Abdeckung, in der die Filterkassette gegen die Vorspannkraft der Feder axial bewegbar aufgenommen ist.

Die Abdeckung ist dabei derart ausgebildet, dass sie Öffnungen aufweist, welche einen Gasaustausch ermöglicht. Die Öffnungen sind dabei andererseits so klein dimensioniert, dass sie zuverlässig die in der Abdeckung befindliche Filterkassette vor Beschädigungen, z.B. durch in einem Sterilbehälter aufgenommene Gegenstände, schützt.

Gemäß einem Aspekt der Erfindung kann die Feder als eine Art Blatt- oder Tellerfeder ausgebildet sein.

Dadurch ist es möglich, die Federkraft nahe der Dichtungsstelle in das System, genauer gesagt in die Filterkassette, einzuleiten.

Gemäß einem Aspekt der Erfindung kann die Feder an der Abdeckung befestigt sein.

Gemäß einem Aspekt der Erfindung kann die Feder mittels eines zentralen Pins an der Abdeckung befestigt sein.

Somit ist es möglich, dass beim Demontieren des Filtersystems, z.B. zur Kontrolle oder zum Austausch der Filterkassette, die Feder gemeinsam mit der Abdeckung abgenommen wird. Es liegen somit beim Abnehmen keine verlierbaren Einzelteile vor.

Gemäß einem Aspekt der Erfindung kann sich die Feder an der Abdeckung und der Filterkassette abstützen.

Gemäß einem Aspekt der Erfindung kann sich die Feder in der Mitte der Abdeckung und der Filterkassette abstützen.

Gemäß einem Aspekt der Erfindung können die Höhen der entlasteten Feder und der Filterkassette zusammen größer sein als die Höhe der Abdeckung.

Mit anderen Worten bedeutet dies, dass bei der Montage der Abdeckung an dem Sterilbehälterwandungsabschnitt die Feder komprimiert werden muss, um somit ihr Höhe zu reduzieren. Dadurch werden die Höhen der nun vorgespannten Feder und der Filterkassette zusammen kleiner oder gleich der Höhe der Abdeckung und die Abdeckung kann arretiert werden. Folglich wirkt bei montierter Abdeckung eine Vorspannkraft der Feder in Richtung des Sterilbehälterwandungsabschnitts auf die Filterkassette. Somit wird zum Einen die sichere Anpressung der Filterkassette inklusive Dichtung gewährleistet und zum Anderen wird die Federkraft Zentral (in der Mitte der Abdeckung) abgestützt. Außerdem ist so ein sicheres Zuhalten der Filterkassette gewährleistet.

Gemäß einem Aspekt der Erfindung kann die Abdeckung speichenradförmig ausgebildet sein.

Gemäß einem Aspekt der Erfindung kann die Abdeckung aus Aluminium gefertigt sein.

Die Kraftaufnahme erfordert, dass die Abdeckung sehr steif gestaltet wird. Dem gegenüber steht die Anforderung, dass das Filtersystem sehr leicht gestaltet sein muss, damit die maximale Beladung des Behälters mit Sterilgut so wenig wie möglich eingeschränkt wird. Durch den bionischen Ansatz, dass das Design der Abdeckung an der Gestalt der Kieselalge angelehnt ist, wird eine innovative Leichbaustruktur offenbart, mit welcher es möglich ist bei gleichbleibenden mechanischen Eigenschaften das Gewicht zu reduzieren. Zusätzlich können durch die Verwendung von Aluminium Vorteile bei der Trocknung durch die Reduktion des Kunststoffanteils erreicht werden.

Weiterhin ist es vorteilhaft, dass durch das relativ geringe Gewicht der Filterkassette und das Wirkprinzip des gewählten Konzepts, das Filtersystem eine vergleichbar geringe, bewegliche träge Masse hat und somit die Sicherheit beim Transport erhöht wird. Mit anderen Worten ist aufgrund der geringen Masse die auf die Filterkassette wirkende Beschleunigung gering, sodass es unwahrscheinlich ist, dass sich die Filterkassette entgegen der Vorspannkraft der Feder bewegt.

Ein Sterilbehälterdeckel des erfindungsgemäßen Sterilbehälters enthält ein erfindungsgemäßes Filtersystem.

Gemäß einem Aspekt der Erfindung kann die Abdeckung an dem Sterilbehälterdeckel arretiert werden.

Gemäß einem Aspekt der Erfindung kann die Abdeckung mittels Bajonettverschluss an dem Sterilbehälterdeckel arretiert werden.

Gemäß einem Aspekt der Erfindung sind als Gegenkontur für den Bajonettverschluss Arretierungsnieten am Sterilbehälterdeckel vorgesehen.

Die Abdeckung übernimmt somit die Arretierung des Gesamtsystems und außerdem wird beim Arretieren der Abdeckung die Feder durch die Filterkassette zwischen der Abdeckung und dem Sterilbehälterdeckel vorgespannt. Als Gegenkontur für den Bajonettverschluss dienen lediglich Arretierungsnieten im Sterilbehälterdeckel und der Bajonettverschluss kann somit nicht falsch angesetzt und in der falschen Position verriegelt werden. Die von der Abdeckung aufgenommenen Federkräfte werden über die Arretierungsnieten in die Deckelstruktur weitergeleitet. Durch diese einfache und sichere Befestigung der Abdeckung und damit des gesamten Filtersystems am Sterilbehälterdeckel wird ein unkompliziertes Handling gewährleistet. Außerdem kann durch die einfache Demontage der Abdeckung eine beidseitige Kontrolle der Filterkassette schnell und problemlos erfolgen.

Der erfindungsgemäße Sterilbehälter, insbesondere zur Aufnahme und sterilen Aufbewahrung von chirurgischen Besteck oder chirurgischen Material, enthält einen durch einen Behälterboden und Behälterwänden gebildeten Aufnahmeraum und einen erfindungsgemäßen Sterilbehälterdeckel zum Verschließen des Aufnahmeraums.

Gemäß einem Aspekt der Erfindung kann das Filtersystem einen sterilen Strömungspfad zwischen dem Behälter-Äußeren und dem Behälter-Inneren gewährleisten.

Durch das in dem Sterilbehälterdeckel vorgesehene Filtersystem und dem dadurch dauerhaft ausgebildeten Strömungspfad ist es möglich, einen sterilen Druckausgleich zwischen dem Behälter-Äußeren und dem Behälter-Inneren zu ermöglichen, ohne das Sterilgut im Behälter zu rekontaminieren. Es wird somit eine Sterilbarriere bzw. ein Sterilbarrieresystem ausgebildet.

Gemäß einem Aspekt der Erfindung kann das Filtersystem zusätzlich eine (Einlass-) Ventilfunktion realisieren, sodass bei Erreichen einer kritischen Druckdifferenz zwischen dem Behälter-Äußeren und dem Behälter-Inneren ein weiterer Strömungspfad freigegeben wird.

Gemäß einem Aspekt der Erfindung kann die Feder eine Vorspannkraft auf die Filterkassette übertragen, sodass die Filterkassette gegen den Sterilbehälterwandungsabschnitt gedrückt wird, und wobei bei Erreichen oder Überschreiten der kritischen Druckdifferenz die von Außen auf die Filterkassette entgegengesetzt der Vorspannkraft der Feder wirkende Kraft größer als die Vorspannkraft der Feder wird, sodass die Filterkassette entgegen der Vorspannkraft der Feder von dem Sterilbehälterwandungsabschnitt zwanghaft gelöst wird.

Mittels dieser Einlassfunktion für hohe Druckänderungsraten können die Dampfsterilisation ermöglicht und der Behälter vor Beschädigungen geschützt werden. So hebt sich bei Erreichen einer kritischen Druckdifferenz zwischen dem Behälter-Äußeren und dem Behälter-Inneren die Filterkassette mit Dichtung, welche von der Feder gegen den Sterilbehälterdeckel gepresst wird, von dem Sterilbehälterdeckel ab und gibt einen weiteren Strömungspfad frei. Die Druckdifferenz, welche zu einer Öffnung des zusätzlichen Strömungspfades führt, ist dabei so hoch ausgelegt, dass die Ventilfunktion nur bei Druckänderungsraten, wie sie bei einem Sterilisationsprozess auftreten, ausgelöst wird.

Zudem wird durch den Einsatz einer Zentrierung mittels Kegel, anstatt einer linearen Führung, ein Verkanten der Filterkassette und somit eine Blockierung der Ventilfunktion verhindert. Weiterhin ist der Kegelsitz nicht selbsthemmend ausgeführt und ein Aufsetzen der Filterkassette beim Schließen der Ventilfunktion an der falschen Position wird verhindert.

### Kurze Beschreibung der Zeichnungen

Fig. 1 zeigt den Aufbau einer Filterkassette und ihre Positionierung in einem Sterilbehälterdeckel.
Fig. 2 zeigt eine Draufsicht der Filterkassette.
Fig. 3 zeigt den Aufbau eines Filtersystems und seine Befestigung in einem Sterilbehälterdeckel.
Fig. 4 zeigt eine perspektivische Ansicht einer Abdeckung.
Fig. 5 zeigt eine perspektivische Ansicht einer Feder.

### Detaillierte Beschreibung bevorzugter Ausführungsformen

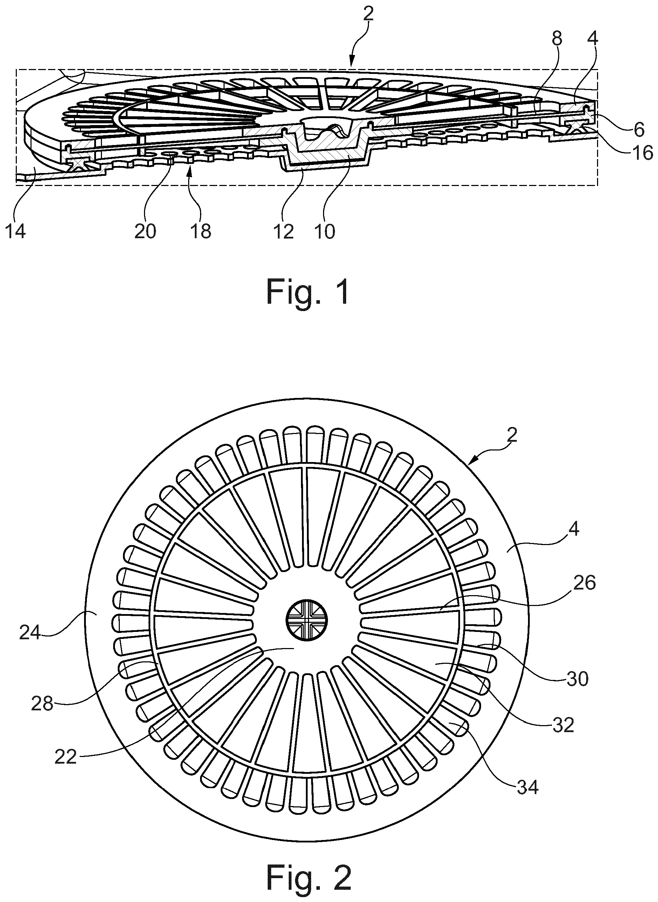

Mit Bezug auf die Fign. 1 und 2 wird nachfolgend eine erfindungsgemäße Filterkassette 2 beschrieben.

Fig. 1 zeigt den Aufbau einer Filterkassette 2 mit einer oberen Klemmscheibe 4 und einer unteren Klemmscheibe 6. Zwischen der oberen Klemmscheibe 4 und der unteren Klemmscheibe 6 ist ein scheibenförmiges Filterelement 8 angeordnet, genauer gesagt eingespannt oder eingepresst. In der Mitte der unteren Klemmscheibe 6 ist ein kegelförmiger Vorsprung 10 vorgesehen, welcher komplementär zu einer - ebenfalls mittig bzw. zentral angeordneten - kegelförmigen Aufnahme 12 in einem Sterilbehälterdeckel 14, als ein Beispiel eines Sterilbehälterwandungsabschnitts, ausgebildet ist. Die obere Klemmscheibe 4 folgt der Kontur der unteren Klemmscheibe 6. Am äußeren Rand der unteren Klemmscheibe 6 ist auf der dem Sterilbehälterdeckel 14 zugewandeten Seite ein Dichtring 16 vorgesehen. Wird die Filterkassette 2 richtig herum, d.h. die untere Klemmscheibe 6 ist dem Sterilbehälterdeckel 14 zugewandt, in den Sterilbehälterdeckel 14 eingesetzt, gleitet der kegelförmige Vorsprung 10 in die kegelförmige Aufnahme 12 und die Filterkassette 2 wird relativ zu dem Sterilbehälterdeckel 14 ausgerichtet bzw. in ihm zentriert. Aufgrund des Kegelsitzes zwischen Filterkassette 2 und Sterilbehälterdeckel 14 gibt es nur eine eindeutige Position, in der die Filterkassette 2 eingesetzt werden kann, und die Filterkassette 2 kann beim Einsetzen bzw. beim Hineingleiten nicht verklemmen. Dabei kommt der Dichtring 16 auf dem Sterilbehälterdeckel 14 derart zum Aufliegen, dass eine in dem Sterilbehälterdeckel 14 vorgesehene Gasaustauschöffnung 18 mit mehreren kreisförmigen Öffnungen 20 vollständig von dem Dichtring 16 in der Umfangsrichtung umschlossen wird. Der Vorsprung 10 und die Aufnahme 12 sind jeweils kegelförmig ausgebildet. Im Grunde genommen ist es jedoch ausreichend, wenn nur eines von beiden, d.h. die Klemmscheibe 6 oder der Sterilbehälterdeckel 14, kegelförmig ausgebildet ist oder eine mittig zulaufende Abgleit- oder Anlagefläche aufweist, welche bei einer Rückstellung von einer ausgelenkten Stellung in deren Anlageposition die Filterkassette 2 zentral zur Gasaustauschöffnung 18 positioniert.

Fig. 2 ist eine Draufsicht der Filterkassette 2, in der das Design der oberen Klemmscheibe 4 dargestellt ist. Von einem inneren Ring 22 erstrecken sich zu einem äußeren Ring 24 mehrere gleichmäßig in der Umfangsrichtung verteilte erste Stege 26. Außerdem ist zwischen dem inneren Ring 22 und dem äußeren Ring 24 ein Zwischenring 28 ausgebildet. Von dem Zwischenring 28 erstrecken sich zu dem äußeren Ring 24 mehrere gleichmäßig zwischen den ersten Stegen 26 in der Umfangsrichtung verteilte zweite Stege 30. Dadurch ergeben sich mehrere erste Öffnungen 32 zwischen dem Innenring 22 und dem Zwischenring 28 und mehrere zweite Öffnungen 34 zwischen dem Zwischenring 28 und dem äußeren Ring 24. Die untere Klemmscheibe 6 ist entsprechend der oberen Klemmscheibe ausgebildet. Durch diese Gestalt der Klemmscheiben 4 und 6 wird erreicht, dass die ersten Öffnungen 32 und die zweiten Öffnungen 34 eine relativ große Fläche ausbilden, damit ein durch die Filterkassette 2 strömendes Gas möglichst wenig durch die Klemmscheiben 4 und 6 behindert wird. Zusätzlich bewirken die ersten und zweiten Stege 26 und 30, sowie der Zwischenring 28, eine stabile und biegesteife Struktur, welche die auf die Filterkassette 2 wirkenden Kräfte aufnimmt und gleichmäßig verteilt.

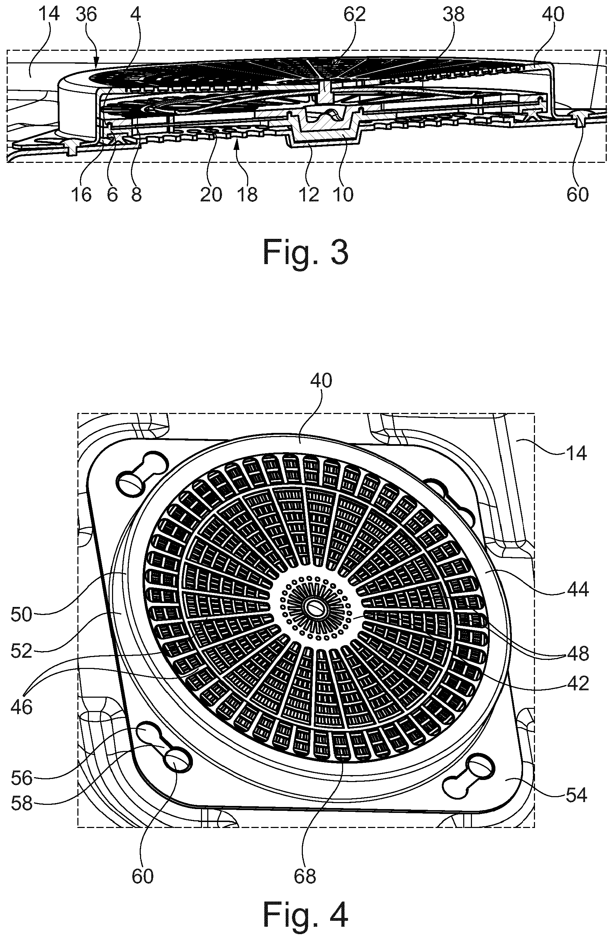

Nachfolgend werden mit Bezug auf die Fign. 3 und 4 ein erfindungsgemäßes Filtersystem 36, sowie seine Befestigung an dem Sterilbehälterdeckel 14 beschrieben. Anschließend wird die Funktionsweise des in einem Sterilbehälter vorgesehenen Filtersystems 36 erläutert.

Das in Fig. 3 dargestellte Filtersystem 36 besteht aus der Filterkassette 2, einer Feder 38 und einer Abdeckung 40. Die Filterkassette 2 ist dabei gegen die Vorspannkraft der Feder 38 axial beweglich in der Abdeckung 40 aufgenommen. Die Gestalt der Abdeckung 40 wird in Fig. 4 gezeigt. Die Abdeckung 40 ist ähnlich der in Fig. 2 gezeigten oberen Klemmscheibe 4 ausgebildet. Von einem Abdeckungsinnenring 42 erstrecken sich zu einem Abdeckungsaußenring 44 mehrere gleichmäßig in der Umfangsrichtung verteilte Abdeckungsstege 46. Zusätzlich sind zwischen dem Abdeckungsinnenring 42 und dem Abdeckungsaußenring 44 mehrere Abdeckungszwischenringe 48 ausgebildet. An der Außenkante 50 des Abdeckungsaußenrings 44 erstreckt sich eine Seitenwand 52. Am anderen, der Außenkante 50 abgewandten Ende der Seitenwand 52 sind vier Flansche 54 ausgebildet, sodass die Abdeckung 40 in der Draufsicht eine quadratische Form hat. In jedem Flansch ist eine Arretierungsöffnung 56 vorgesehen, an welche sich ein Schlitz 58 anschließt, dessen Breite kleiner ist als der Durchmesser der Arretierungsöffnung 56. Die Arretierungsöffnungen 56 sind dabei so dimensioniert, dass an dem Sterilbehälterdeckel 14 befestigte Arretierungsnieten 60 durch die Arretierungsöffnung 56 geführt werden können. Eine anschließende Drehung der Abdeckung 40 bewirkt, dass die Arretierungsnieten 60 in die Schlitze 58 geschoben werden und die Abdeckung 40 fest mit dem Sterilbehälterdeckel 14 verbunden wird.

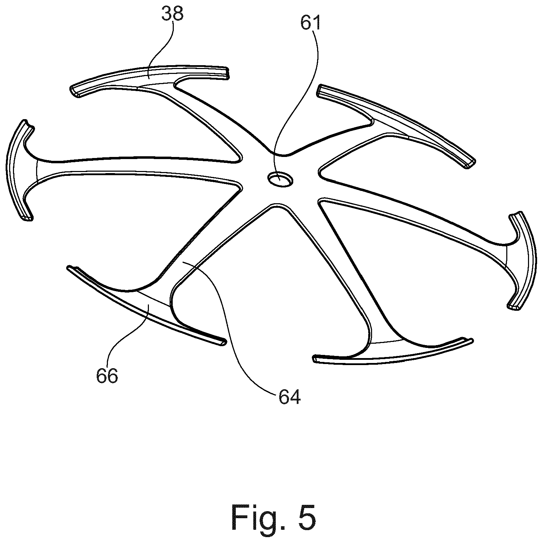

Fig.5 zeigt die als Tellerfeder speichenartig ausgebildete Feder 38. Von einer zentralen Befestigungsöffnung 61, in welche ein in Fig. 3 dargestellter zentraler Pin 62 zur Befestigung der Feder 38 an der Abdeckung 40 eingeführt werden kann, erstrecken sich in radialer Richtung nach Außen mehrere Speichen 64, deren radial äußeres Ende 66 T-förmig ausgebildet ist. Mit anderen Worten sind die Speichen 64 in der Umfangsrichtung an ihrem radial äußeren Ende 66 aufgeweitet. Somit ist es möglich einen Anpressdruck in Umfangsrichtung gleichmäßig zu übertragen.

Bei dem in Fig. 3 dargestellten Filtersystem 36 ist die Feder 38 mittels des Pins 62 in der Mitte der Abdeckung 40 an der dem Sterilbehälterdeckel 14 zugewandten Seite der Abdeckung 40 befestigt. Die Filterkassette 2 ist zwischen der Abdeckung 40 mit der Feder 38 und dem Sterilbehälterdeckel 14 positioniert, sodass sich die Feder 38 an der Abdeckung 40 und der Filterkassette 2, genauer gesagt der oberen Klemmscheibe 4, abstützt. Da die Feder 38 als Tellerfeder ausgeführt ist, stützt sich die Feder 38 im äußeren Bereich der Filterkassette 2 ab, was dazu führt, dass die Federkraft in der Nähe des Dichtrings 16 in die Filterkassette 2 eingeleitet wird. Dadurch kommt der Dichtring 16 in Umfangsrichtung gleichmäßig sicher an dem Sterilbehälterdeckel 14 zum Anliegen und stellt somit zuverlässig eine Dichtung her. Mit anderen Worten wird vorteilhafterweise über die in Umfangsrichtung gleichmäßig Vorspannkraft der Feder 38 stets eine in Umfangsrichtung gleichmäßige Andrückkraft des Dichtrings 16 gewährleistet. Die obere Klemmscheibe 4 und die untere Klemmscheibe 6 sind in ihrer Gestaltung derart angepasst, sodass sie im Bereich der Krafteinleitung und Übertragung, d.h. im äußeren Bereich, zusätzlich durch den Zwischenring 28 und die zweiten Stege 30 verstärkt sind. Da die Federkraft zentral in der Abdeckung 40 aufgenommen und von ihr über die Arretierungsnieten 60 in den Sterilbehälterdeckel 14 weitergeleitet wird, ist die Abdeckung 40 entlang der gesamten radialen Ausdehnung zwischen dem Abdeckungsinnenring 42 und dem Abdeckungsaußenring 46 mit zahlreichen Abdeckungsstegen 46 und Abdeckungszwischenringen 48 verstärkt. Dadurch wird auch eine Vielzahl kleiner Abdeckungsöffnungen 68 in der Abdeckung 40 ausgebildet. Diese ermöglichen einen Gasaustausch und schützen die Filterkassette 2 zusätzlich vor mechanischen Beschädigungen.

Das vorstehend beschriebene Filtersystem 36 wird in einem Sterilbehälterdeckel 14 befestigt, welcher wiederum zum Verschließen eines (nicht dargestellten) Sterilbehälters dient. Die Montage des Filtersystems 36 erfolgt dabei in nur zwei einfachen Schritten. Zuerst wird die Filterkassette 2 bzw. der kegelförmige Vorsprung 10 der unteren Klemmscheibe 6 in den Sterilbehälterdeckel 14 bzw. die kegelförmige Aufnahme 12 eingesetzt. Anschließend werden die Arretierungsöffnungen 56 der Abdeckung 40 über die Arretierungsnieten 60 geführt und die Abdeckung 40 wird nur durch eine kleine Drehung, d.h. nach dem Prinzip eines Bajonettverschlusses, sicher arretiert. Dabei wird die Filterkassette 2 über die in der Abdeckung 40 befestigte Feder 38 in ihrer Position fixiert. Dabei entsteht keine feste Verbindung zwischen der Feder 38 und der Filterkassette 2, was die Montage vereinfacht. Mit anderen Worten gleiten die Feder 38 und die Filterkassette 2 aneinander ab und die Feder 38 wird beim Arretieren der Abdeckung 40 durch die Abdeckung 40 und die Filterkassette 2 vorgespannt. Diese Tatsache ist in Bezug auf die Prozesssicherheit von besonderem Vorteil, da bei einer Montage oder einer Wiedermontage kein Bauteil vergessen werden kann. Der Grund dafür ist die haptische Rückmeldung, welche der Anwender erfährt, wenn das System vollständig montiert wurde. Ohne die Abdeckung 40 kann die Filterkassette 2 nicht arretiert werden und ohne die Filterkassette 2 wird die Feder 38 nicht vorgespannt und der Anwender erfährt keine haptische Rückmeldung beim Arretieren der Abdeckung 40. Mit anderen Worten wird ein Fehler beim Zusammenbau erkannt, wenn die Abdeckung 40 nicht entgegen der Vorspannkraft der Feder 38 gegen den Sterilbehälterdeckel 14 gedrückt werden muss.

Weiterhin sei erwähnt, dass die Filterkassette 2 sehr einfach ersetzt oder beidseitig kontrolliert werden kann, da lediglich durch eine leichte Drehung der Bajonettverschluss der Abdeckung 40 geöffnet und diese entfernt werden muss. Zusätzlich ist eine Reinigung so sehr einfach möglich. Außerdem ist das gesamte Filtersystem 36 sehr einfach aufgebaut, da es im Wesentlichen nur aus zwei Teilen besteht, der Filterkassette 2 und der Abdeckung 40 mit der darin befestigten Feder 38. Dadurch liegen zu keinem Zeitpunkt Kleinteile oder einzelne Bauteile vor, was das Handling vereinfacht. Zudem wird aufgrund der geringen Anzahl der Komponenten und der genutzten Leichtbaukonzepte das Gesamtgewicht des Filtersystems reduziert. Gerade aufgrund der geringen, beweglichen trägen Masse kann die Sterilität auch bei Transporten, trotz der dort wirkenden Beschleunigungen, mit hoher Sicherheit gewährleistet werden.

In einem normalen Betriebszustand des Sterilbehälters, d.h. wenn eine Druckdifferenz zwischen dem Behälter-Äußeren und dem Behälter-Inneren kleiner als eine vorbestimmte kritische Druckdifferenz ist, drückt die Feder 38 die Filterkassette 2, insbesondere den Dichtring 16, gegen den Sterilbehälterdeckel 14. Dadurch wird ein steriler Strömungspfad von dem Behälter-Äußeren, über die Gasaustauschöffnung 18 in dem Sterilbehälterdeckel 14, die Filterkassette 2 und die Abdeckung 40, in das Behälter-Innere, oder umgekehrt, erzeugt. Durch das lineare Andrücken der Filterkassette 2 durch die als Tellerfeder ausgeführte Feder 38 und die Verwendung eines Kegelsitzes, welcher keine durchgängige Bohrung in der Filterkassette für die Zentrierung benötigt, ist somit nur eine Dichtung erforderlich, um eine Sterilbarriere sicherzustellen. Außerdem kann eine Dichtung mit großer Kompressionshöhe verwendet werden, wodurch größere Produktionstoleranzen ausgeglichen werden können.

Steigt jedoch, z.B. im Falle eines Sterilisationsvorgangs, der Druck außerhalb des Behälters an und überschreitet somit die Druckdifferenz zwischen dem Behälter-Äußerem und dem Behälter-Innerem die vorbestimmte kritische Druckdifferenz, so wird die Filterkassette 2 entgegen der Vorspannkraft der Feder 38 in Richtung der Abdeckung 40 gedrückt. Dadurch hebt sich die Filterkassette 2 von dem Sterilbehälterdeckel 14 ab und der Dichtring 16 kommt nicht mehr, oder nicht mehr ausreichend für eine sichere Dichtung, mit dem Sterilbehälterdeckel 14 in Kontakt. Dadurch entsteht ein zweiter, unsteriler Strömungspfad von dem Behälter-Äußeren, über die Gasaustauschöffnung 18 in dem Sterilbehälterdeckel 14, zwischen der Filterkassette 2 und der Seitenwand 52 der Abdeckung 40 hindurch und durch die Abdeckung 40 in das Behälter-Innere. Dieser zweite Strömungspfad ist notwendig, damit der Druckausgleich bei einer großen Druckdifferenz schneller erfolgen kann und der Sterilbehälter somit vor einer Beschädigung geschützt wird. Die durch das Filterelement 8 begrenzte Durchflussrate des ersten, sterilen Strömungspfades wäre dafür nicht ausreichend.

Sobald sich die Drücke innerhalb und außerhalb des Behälters wieder aneinander angeglichen haben, d.h. wenn die Druckdifferenz kleiner als die kritische Druckdifferenz wird, drückt die Feder 38 die Filterkassette 2 in Richtung des Sterilbehälterdeckels 14. Dabei gleitet der kegelförmige Vorsprung 10 der unteren Klemmscheibe 6 in die kegelförmige Aufnahme 12 des Sterilbehälterdeckels 14. Dadurch wird die Filterkassette 2 beim Zurückdrücken durch die Feder 38 sicher geführt und automatisch in dem Sterilbehälterdeckel 14 zentriert. Aufgrund der Kegelform, bzw. dem somit ausgebildeten Kegelsitz, kann sich die Filterkassette 2 nicht verklemmen und die Ventilfunktion des Filtersystems 36 kann sichergestellt werden, d.h. ein Blockieren der Ventilfunktion wird ausgeschlossen. Die Zentrierung der Filterkassette 2 im Sterilbehälterdeckel 14 ist besonders wichtig, damit der Dichtring 16 die im Sterilbehälterdeckel 14 ausgebildete Gasaustauschöffnung 18 sicher umschließt und somit der Gasaustausch durch das Filtersystem 36 nur über den sterilen Strömungspfad möglich ist.

Neben der vorstehend beschriebenen Ausführungsform sind auch noch alternative Ausgestaltungen der Filterkassette 2, des Filtersystems 36, des Sterilbehälterdeckels 14 und des Sterilbehälters möglich. So kann beispielsweise an der unteren Klemmscheibe 6 eine kegelförmige Aufnahme vorgesehen sein, welche komplementär zu einem an dem Sterilbehälterdeckel 14 vorgesehenen kegelförmigen Vorsprung ausgebildet ist. Außerdem kann beispielsweise die Feder 38 an der oberen Klemmscheibe 4 der Filterkassette 2 befestigt sein und sich an der der Filterkassette 2 zugewandeten Seite der Abdeckung 40 abstützen.

## Patentansprüche

1. Sterilbehälter mit einer gasdurchlässigen Filterkassette (2), die ein in der Filterkassette (2) angeordnetes Filterelement (8) aufweist, wobei zum Zentrieren der Filterkassette (2) bezüglich eines Sterilbehälterwandungsabschnitts (14), der zumindest eine Gasaustauschöffnung (18) aufweist, an der Filterkassette (2) ein erstes, kegelförmiges Zentriermittel (10) vorgesehen ist, welches mit einem an dem Sterilbehälterwandungsabschnitt (14) vorgesehenen zweiten Zentriermittel (12) nichtselbsthemmend zusammenwirkt, wenn die Filterkassette (2) in dem Sterilbehälterwandungsabschnitt (14) eingesetzt oder an diesem angeordnet ist.

2. Sterilbehälter gemäß Anspruch 1, wobei ein kegelförmiger Vorsprung (10) oder eine kegelförmige Aufnahme als das erste Zentriermittel an der Filterkassette (2) vorgesehen ist, so dass diese mit einer entsprechend komplementär ausgebildeten Aufnahme (12) oder einem entsprechend komplementär ausgebildeten Vorsprung als zweites Zentriermittel an dem Sterilbehälterwandungsabschnitt (14) einen nicht-selbsthemmenden Kegelsitz bildet, wenn die Filterkassette (2) in dem Sterilbehälterwandungsabschnitt (14) eingesetzt oder an diesem angeordnet ist.

3. Sterilbehälter gemäß Anspruch 1 oder 2, wobei die Filterkassette (2) aus einer oberen Klemmscheibe (4) und einer unteren Klemmscheibe (6) ausgebildet ist und das Filterelement (8) vorzugsweise zwischen der oberen Klemmscheibe (4) und der unteren Klemmscheibe (6) angeordnet ist.

4. Sterilbehälter gemäß einem der vorstehenden Ansprüche, wobei ein Dichtring (16) an der Filterkassette (2) vorgesehen ist und der Dichtring (16) vorzugsweise an der dem Sterilbehälterwandungsabschnitt (14) zugewandten Seite der Filterkassette (2) und besonders bevorzugt in einem äußeren Randbereich der Filterkassette (2) vorgesehen ist.

5. Sterilbehälter gemäß einem der vorstehenden Ansprüche, wobei der Sterilbehälter weiter ein Filtersystem (36) mit der gasdurchlässigen Filterkassette (2), einer vorzugsweise als eine Art Blatt- oder Tellerfeder ausgebildeten Feder (38) und einer an dem Sterilbehälterwandungsabschnitt (14) montierbaren Abdeckung (40), in der die Filterkassette (2) gegen die Vorspannkraft der Feder (38) axial bewegbar aufnehmbar ist, aufweist.

6. Sterilbehälter gemäß Anspruch 5, wobei die Feder (38) vorzugsweise mittels eines zentralen Pins (62) an der Abdeckung (40) befestigt ist.

7. Sterilbehälter gemäß Anspruch 5 oder 6, wobei sich die Feder (38) vorzugsweise in der Mitte der Abdeckung (40) und an der Filterkassette (2) abstützt.

8. Sterilbehälter nach einem der Ansprüche 5 bis 7, wobei der Sterilbehälter weiter einen Sterilbehälterdeckel (14) mit dem Filtersystem (36) aufweist und die Abdeckung (40) vorzugsweise mittels Bajonettverschluss an dem Sterilbehälterdeckel (14) arretierbar ist.

9. Sterilbehälter nach Anspruch 8, insbesondere zur Aufnahme und sterilen Aufbewahrung von chirurgischen Besteck oder chirurgischen Material, wobei der Sterilbehälter weiter einen durch einen Behälterboden und Behälterwänden gebildeten Aufnahmeraum aufweist und der Sterilbehälterdeckel (14) zum Verschließen des Aufnahmeraums vorgesehen ist.

10. Sterilbehälter gemäß Anspruch 9, wobei das Filtersystem (36) zusätzlich eine (Einlass-) Ventilfunktion realisiert, sodass bei Erreichen einer kritischen Druckdifferenz zwischen dem Behälter-Äußeren und dem Behälter-Inneren ein weiterer Strömungspfad freigegeben wird.

11. Sterilbehälter gemäß Anspruch 10, wobei die Feder (38) eine Vorspannkraft auf die Filterkassette (2) überträgt, sodass die Filterkassette (2) gegen den Sterilbehälterwandungsabschnitt (14) gedrückt wird, und wobei bei Erreichen oder Überschreiten der kritischen Druckdifferenz die von Außen auf die Filterkassette (2) entgegengesetzt der Vorspannkraft der Feder (38) wirkende Kraft größer als die Vorspannkraft der Feder (38) wird, sodass die Filterkassette (2) entgegen der Vorspannkraft der Feder (38) von dem Sterilbehälterwandungsabschnitt (14) zwanghaft gelöst wird.

## Claims

1. Sterile container with a gas-permeable filter cassette (2), comprising a filter element (8) arranged in the filter cassette (2), wherein for centering the filter cassette (2) with regard to a sterile container wall section (14) having at least one gas exchange opening (18), a first, conical centering means (10) is provided on the filter cassette (2) and cooperates in a non-self-retaining manner with a second centering means (12) provided on the sterile container wall section (14) when the filter cassette (2) is inserted in or arranged on the sterile container wall section (14).

2. Sterile container according to claim 1, wherein a conical projection (10) or a conical seating is provided as the first centering means on the filter cassette (2), so that it forms a non-self-restraining conical seat with a correspondingly complementary seating (12) or a correspondingly complementary projection as a second centering means on the sterile container wall section (14) when the filter cassette (2) is inserted in or arranged on the sterile container wall section (14).

3. Sterile container according to claim 1 or 2, wherein the filter cassette (2) is formed from an upper clamping disc (4) and a lower clamping disc (6), and the filter element (8) is preferably arranged between the upper clamping disc (4) and the lower clamping disc (6).

4. Sterile container according to one of the preceding claims, wherein a sealing ring (16) is provided on the filter cassette (2), the sealing ring (16) being preferably provided on the side of the filter cassette (2) facing the sterile container wall section (14) and particularly preferably in an outer peripheral region of the filter cassette (2).

5. Sterile container according to one of the preceding claims, wherein the sterile container comprises a filter system (36) comprising the gas-permeable filter cassette (2), a spring (38) preferably realized in the form of a leaf spring or disc spring, and a cover (40) which can be mounted on the sterile container wall section (14) and in which the filter cassette (2) can be received so as to be axially movable against the prestressing force of the spring (38).

6. Sterile container according to claim 5, wherein the spring (38) is fastened to the cover (40) preferably by a central pin (62).

7. Sterile container according to claim 5 or 6, wherein the spring (38) is supported preferably in the center of the cover (40) and on the filter cassette (2).

8. Sterile container according to one of claims 5 to 7, wherein the sterile container further comprises a sterile container lid (14) comprising the filter system (36), and the cover (40) can be locked to the sterile container lid (14) preferably by means of a bayonet lock.

9. Sterile container according to claim 8, in particular for receiving and sterile storage of surgical instruments or surgical material, wherein the sterile container further comprises a receiving space formed by a container bottom and container walls and the sterile container lid (14) is provided for closing the receiving space.

10. Sterile container according to claim 9, wherein the filter system (36) additionally realizes an (inlet) valve function so that, when a critical pressure difference between the container exterior and the container interior is reached, a further flow path is opened.

11. Sterile container according to claim 10, wherein the spring (38) transfers a prestressing force to the filter cassette (2) so that the filter cassette (2) is pressed against the sterile container wall section (14), and wherein, when the critical pressure difference is reached or exceeded, the force acting on the filter cassette (2) from the outside opposed to the prestressing force of the spring (38) becomes greater than the prestressing force of the spring (38), so that the filter cassette (2) is forcefully opened from the sterile container wall section (14) against the prestressing force of the spring (38).

## Revendications

1. Récipient stérile avec une cassette filtrante perméable aux gaz (2) qui présente un élément filtrant (8) disposé dans la cassette filtrante (2), dans lequel pour le centrage de la cassette filtrante (2) par rapport à une section de paroi de récipient stérile (14) qui présente au moins une ouverture d'échange de gaz (18), un premier moyen de centrage conique (10) est prévu au niveau de la cassette filtrante (2), lequel coopère de manière non-autobloquante avec un second moyen de centrage (12) prévu au niveau de la section de paroi de récipient stérile (14) lorsque la cassette filtrante (2) est employée dans la section de paroi de récipient stérile (14) ou disposée au niveau de celle-ci.

2. Récipient stérile selon la revendication 1, dans lequel une saillie conique (10) ou une réception conique est prévue en tant que premier moyen de centrage au niveau de la cassette filtrante (2) de sorte qu'elle forme avec une réception (12) réalisée adéquatement de manière complémentaire ou avec une saillie réalisée adéquatement de manière complémentaire en tant que second moyen de centrage au niveau de la section de paroi de récipient stérile (14) un siège conique non-autobloquant lorsque la cassette filtrante (2) est employée dans la section de paroi de récipient stérile (14) ou disposée au niveau de celle-ci.

3. Récipient stérile selon la revendication 1 ou 2, dans lequel la cassette filtrante (2) est réalisée à partir d'une rondelle de serrage supérieure (4) et d'une rondelle de serrage inférieure (6) et l'élément filtrant (8) est de préférence disposé entre la rondelle de serrage supérieure (4) et la rondelle de serrage inférieure (6).

4. Récipient stérile selon l'une quelconque des revendications précédentes, dans lequel une bague d'étanchéité (16) est prévue au niveau de la cassette filtrante (2) et la bague d'étanchéité (16) est prévue de préférence au niveau de la face de la cassette filtrante (2) tournée vers la section de paroi de récipient stérile (14) et le plus préférentiellement dans une zone en bordure externe de la cassette filtrante (2).

5. Récipient stérile selon l'une quelconque des revendications précédentes, dans lequel le récipient stérile présente en outre un système filtrant (36) avec la cassette filtrante perméable aux gaz (2), un ressort (38) réalisé de préférence en tant que ressort à lames ou ressort à disque et un capot (40) pouvant être monté au niveau de la section de paroi de récipient stérile (14), dans lequel la cassette filtrante (2) peut être logée de manière mobile axialement à l'encontre de la force de précontrainte du ressort (38).

6. Récipient stérile selon la revendication 5, dans lequel le ressort (38) est de préférence fixé au capot (40) au moyen d'une broche centrale (62).

7. Récipient stérile selon la revendication 5 ou 6, dans lequel le ressort (38) s'appuie de préférence au centre du capot (40) et au niveau de la cassette filtrante (2).

8. Récipient stérile selon l'une quelconque des revendications 5 à 7, dans lequel le récipient stérile présente en outre un couvercle de récipient stérile (14) avec le système filtrant (36) et le capot (40) peut être bloqué de préférence au moyen d'une fermeture à baïonnette au niveau du couvercle de récipient stérile (14).

9. Récipient stérile selon la revendication 8, en particulier pour la réception et la conservation stérile d'instruments chirurgicaux ou de matériel chirurgical, dans lequel le récipient stérile présente en outre un espace de réception formé par le biais d'un fond de récipient et de parois de récipient et le couvercle de récipient stérile (14) est prévu pour la fermeture de l'espace de réception.

10. Récipient stérile selon la revendication 9, dans lequel le système filtrant (36) réalise en outre une fonction d'aération (d'entrée) de sorte que lorsqu'une différence de pression critique entre l'extérieur du récipient et l'intérieur du récipient est atteinte un autre chemin d'écoulement est libéré.

11. Récipient stérile selon la revendication 10, dans lequel le ressort (38) transfère une force de précontrainte sur la cassette filtrante (2) de sorte que la cassette filtrante (2) est poussée contre la section de paroi de récipient stérile (14) et dans lequel lorsque la différence de pression critique est atteinte ou dépassée par le haut la force agissant depuis le haut sur la cassette filtrante (2) à l'opposé de la force de précontrainte du ressort (38) est plus grande que la force de précontrainte du ressort (38) de sorte que la cassette filtrante (2) à l'encontre de la force de précontrainte du ressort (38) est enlevée de manière forcée de la section de paroi de récipient stérile (14).
